# EUROPÄISCHE PATENTSCHRIFT

(11) **EP 2 507 206 B1**
(45) Veröffentlichungstag und Bekanntmachung des Hinweises auf die Patenterteilung: **03.12.2014**
(21) Anmeldenummer: 10790533.3
(22) Anmeldetag: 03.12.2010
(51) Int. Cl.: C07C 263/04, C07C 265/12, C07C 265/14

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 04.12.2009 EP 09178079
(43) Veröffentlichungstag der Anmeldung: 10.10.2012
(73) Patentinhaber: BASF SE, 67056 Ludwigshafen (DE)
(72) Erfinder: MATTKE, Torsten, 67251 Freinsheim (DE); STRÖFER, Eckhard, 68163 Mannheim (DE); LESCHINSKI, Julia, 68161 Mannheim (DE); ABDALLAH, Radwan, 67063 Ludwigshafen (DE); FRANZKE, Axel, 68161 Mannheim (DE); BOCK, Michael, 67152 Ruppertsberg (DE)
(86) Internationale Anmeldenummer: PCT/EP2010/068811
(87) Internationale Veröffentlichungsnummer: WO 2011/067369

(56) Entgegenhaltungen:
- WO-A2-02/24634
- US-A- 4 207 251
- US-A- 5 686 645
- LEARDINI, RINO ET AL: "A new and facile synthesis of alkyl N-arylcarbamates" SYNTHESIS, Bd. 3, 1982, Seiten 225-227, XP002620437 ISSN: 0039-7881

## Beschreibung

Die vorliegende Erfindung betrifft ein neues Verfahren zur Herstellung von Isocyanaten.

Isocyanate, insbesondere Diisocyanate sind wertvolle Grundstoffe für die Herstellung von Polyurethanen. Polyurethane gelten als die Klasse von Kunststoffen mit der höchsten Einsatzbreite in verschiedenen Anwendungen. Entsprechend zeigen die weltweiten Märkte von Diisocyanaten und Polyurethanen seit Jahren hohe Wachstumsraten. Die wichtigsten Isocyanate sind MDI (Methylendiphenyldiisocyanat), TDI (Toluylendiisocyanat) und PMDI (polymeres Methylendiphenyldiisocyanat).

Im TDI Verfahren wird zunächst eine Mischung erhalten, die überwiegend aus Isomeren des TDI besteht und aus Oligomeren, die über Harnstoff- und Diisocyanatgruppen vernetzt sind. Die Zusammensetzung der Oligomeren ist durch den Herstellprozess bestimmt. Die reinen Isomere beziehungsweise Isomerenmischungen werden aus der rohen TDI Mischung im Allgemeinen durch einen destillativen Trennschritt erhalten. Bevorzugte Isomerenmischungen sind solche von 2,4 und 2,6 TDI im Molverhältnis von 80 : 20 und 60 : 40.

Es ist bekannt, Isocyanate aus Aminen und Phosgen herzustellen. Die Umsetzung wird je nach Art der Amine entweder in der Gasphase oder in der flüssigen Phase sowohl diskontinuierlich als auch kontinuierlich durchgeführt (W. Siefken; Liebig Annalen der Chemie 562, 75 (1949)). Die kontinuierliche Herstellung von organischen Diisocyanaten durch Reaktionen von primären organischen Aminen und Phosgen ist vielfach beschrieben und im großtechnischen Maßstab üblich (Ullmanns Enzyklopädie der technischen Chemie, Band 7, dritte Auflage, Carl Hanser Verlag, S. 76 ff. (1993)).

Heutige großtechnische Synthesen von Isocyanaten erfolgen fast ausschließlich in kontinuierlichen Verfahren. In der Regel ist die kontinuierliche Ausführungsform des Verfahrens mehrstufig. In der ersten Stufe der Phosgenierung wird das Amin mit Phosgen zum entsprechenden Carbamoylchlorid und Chlorwasserstoff und zu Aminhydrochloriden umgesetzt.

Die primäre Reaktion zwischen Aminen und Phosgen ist sehr schnell und exotherm. Zwecks Minimierung der Bildung von Nebenprodukten und Feststoffen werden Amin und Phosgen, beide gegebenenfalls in einem organischen Lösungsmittel gelöst, schnell vermischt. Die nächste Stufe der Phosgenierung umfasst sowohl die Zersetzung des Carbamoylchlorid zum gewünschten Isocyanat und Chlorwasserstoff als auch die Phosgenierung des Aminhydrochlorids zum Carbamoylchlorid. Flüssigphasenphosgenierungen sind zum Beispiel beschrieben in EP-A 1 616 857, WO 2004/056756, WO 2006/130405 und EP-A 012 70 544. Zur Vermeidung der Bildung von den unerwünschten Zwischenprodukten der Aminhydrochloride kann die Phosgenierung auch als Gasphasenphosgenierung bei hohen Temperaturen geführt werden. Verfahren sind beispielhaft beschrieben in EP-B 1 449 826 und WO 2008006775 (Aerosolphosgenierung). Auch Phosgenierungen im überkritischen Bereich sind beschrieben worden (WO 2008/049783).

Ein entscheidender Nachteil der oben beschriebenen Verfahren ist der Einsatz von Phosgen, welches eine starke Toxizität für Mensch und Umwelt aufweist. Anlagen, in denen Phosgen verarbeitet wird müssen daher über umfangreiche kostenintensive Sicherheitskonzepte und -einrichtungen verfügen sowie strenge gesetzliche Auflagen erfüllen.

Für Spezialdiisocyanate in kleiner Kapazität sind bereits phosgenfreie Verfahren zur Anwendung gekommen (EP-A 18588, EP-A 27952, EP-A 126299, EP-A 566925). Für aromatische Diisocyanate, die in großen Mengen produziert werden, werden mögliche phosgenfreie Wege in der Literatur diskutiert, sind aber bisher nicht umgesetzt worden (WO 2006/131381, EP-A 1 259 480, EP-A 1 160 239, EP-A 28338, Chinese Journal of Catalysis Vol. 26 No.11 (2005), 987 - 992, CN-A-1850792). Beispielhaft erwähnt seien hier die reduktive Carbonylierung von Nitroverbindungen, die oxidative Carbonylierung von Aminen, die Carboalkoxylierung bzw. Carboaroxylierung von Aminen sowie die Synthese und anschließende Spaltung von Carbamaten (WO 2008/025659 A1; Six und Richter: Isocyanates, Organic in Ullmanns Enzyklopädie der technischen Chemie, Band 7, Carl Hanser Verlag).

Auch die Oxidehydrierung von Formamiden wurde in der Literatur bereits beschrieben. Wegefahrt (Dissertation Ralf Wegefahrt, Universität zu Köln, 2000) beschreibt die Oxidehydrierung von Monomethylformamid mit einem Sauerstoff/Helium Gemisch an einem Silberkatalysator. Nach der Reaktion wird das austretende Reaktionsgemisch mit einer Quenche, befüllt mit Wasser oder Ethanol, schnell auf RT abgekühlt. Zur quantitativen Ermittlung des Reaktionsumsatzes wird das gebildete flüchtige Methylisocyanat durch Zugabe von Methylamin in Form von N,N'-Dimethylharnstoff abgefangen. Von dem Abfangen des Methylisocyanats mit Ethanol als Urethan wurde wegen der geringeren Reaktionsgeschwindigkeit abgesehen.

In der US-A 4,207,251 wird die Oxidehydrierung von aliphatischen und aromatischen Formamiden an verschiedenen Edelmetallen beschrieben. Die Formamide werden gasförmig in Gegenwart von Sauerstoff bei 300 bis 600°C zu den entsprechenden Isocyanaten umgesetzt. Das Reaktionsgemisch wird dann, gegebenenfalls nachvorheriger Kühlung, mit einem mit Wasser nicht mischbaren, hochsiedenden Lösungsmittel (Siedepunkt > 150°C, bevorzugt 200 bis 300°C) für das Isocyanat zur Abtrennung von gebildetem Wasser zusammengebracht. Die Ausbeute an 2,4-Toluoldiisocyanat ist mit 27% gering, da das Formamid teilweise oder vollständig zu CO₂ und Wasser oxidiert oder teilweise bis vollständig zum Amin decarbonyliert wird. Die so entstandenen Amine bilden dann mit den gebildeten Isocyanaten schwersiedende Harnstoffe, welche unter hohem Energieaufwand zurückgespalten werden müssten.

Die Nachteile der beschriebenen phosgenfreien Verfahren liegen neben hohen spezifischen Investitionskosten in der geringeren Selektivität und damit Gesamtausbeute gegenüber der herkömmlichen Variante mit Phosgen begründet. Der Grund für die geringe Ausbeute sind unerwünschte Parallelreaktionen wie die Oxidation oder die Decarbonylierung. Damit sind diese Wege sowohl ökonomisch (Kosten) wie auch ökologisch (mehr Reststoffe und Nebenprodukte) ungünstig und verbesserungsbedürftig.

Aufgabe der vorliegenden Erfindung ist daher die Entwicklung eines verbesserten phosgenfreien Verfahrens zur Herstellung von aromatischen Isocyanaten, welches die vorgenannten Nachteile und insbesondere die unerwünschte Harnstoffbildung nicht aufweist.

Gegenstand der Erfindung ist daher ein Verfahren zur Herstellung von aromatischen Isocyanaten durch Umsetzung der korrespondierenden Formamide mit einem sauerstoffhaltigen Gas an Edelmetallkatalysatoren bei Temperaturen von 300 bis 600 °C und einer Kontaktzeit von 1 bis 1000 ms, gekennzeichnet durch:
a. Verdampfung des Formamids vor Eintritt in die Reaktionszone,
b. Quench der erhaltenen Reaktionsmischung mit einer alkoholhaltigen Quenchflüssigkeit und
c. Spaltung des gebildeten Urethans zu Isocyanat und Alkohol.

Bei den eingesetzten Formamiden handelt es sich um mono- oder polycyclische aromatische Mono- Bis- oder Polyformamide, welche N-monosubstituiert sind. Die Formamide entsprechen der allgemeinen Formel R(NH-CHO)ₙ, worin R für einen gegebenenfalls substituierten C₆-C₂₄-Arylrest, vorzugsweise C₆-C₁₈-Arylrest, insbesondere C₆-C₁₀-Arylrest steht, und besonders bevorzugt Phenyl ist, und n eine ganze Zahl von 1 bis 3, vorzugsweise 1 oder 2, pro aromatischen Cyclus ist. Geeignete Substituenten sind zum Beispiel Chlor, Fluor, Cyano, Alkyl, Alkoxy, Alkylcarbonyl und/oder Alkoxycarbonyl, wobei Alkyl und Alkoxy im Allgemeinen 1 bis 10, vorzugsweise 1 bis 6, besonders bevorzugt 1 bis 4 Kohlenstoffatome, aufweisen.

Bevorzugt sind Phenylformamid (= Formanilid) und Toluolbisformamid, besonders bevorzugt sind 2,4- und 2,6-Toluolbisformamid.

Die dem gewünschten Isocyanat entsprechenden Formamide werden vor Eintritt in die Reaktionszone durch Strippung oder Verdüsen mit den gasförmigen Einsatzstoffen bzw. einem heißen Inertgas- und/oder Sauerstoffgasstrom, mittels eines Wendelrohrverdampfers, mittels eines mikro- oder millistrukturierten Verdampfers, mittels Fallfilmverdampfers oder eines Dünnschichtverdampfers, oder durch Einspritzen auf eine heiße Verdampferschüttung verdampft. Bevorzugt werden die Formamide kontinuierlich aufgeschmolzen und als Schmelze in den Verdampfer transportiert.

Die Verweilzeit in der Verdampfung beträgt 1-1000 ms, bevorzugt 10-400 ms.

Das Formamid wird gasförmig in den Reaktor eingeleitet, wo die Umsetzung mit dem sauerstoffhaltigen Gas an einem Edelmetallkatalysator erfolgt, wobei dieser als Festbett in einem Rohrreaktor aber auch als Wirbelbett in einem Wirbelschichtreaktor eingesetzt werden kann.

Unter für das Verfahren geeigneten Edelmetallkatalysatoren sind die der Metalle Cu, Au, Ag, Re, Pd, Pt, Rh, Ru, W oder Os oder deren Mischungen zu verstehen. Bevorzugt werden als Edelmetallkatalysatoren solche aus Cu und/oder Ag eingesetzt. Besonders bevorzugt ist die Verwendung von Silberkatalysatoren. Ferner kann der Edelmetallkatalysator mit geeigneten Dotierungen von Fremdatomen wie Cu, Ag, Au, Re, Pd und/oder Pt (= Promotoren) versehen sein. Bevorzugt ist der Einsatz von Katalysatoren aus Silber und Promotoren wie Cu, Au, Re, Pd und/oder Pt.

Die vorgenannten Edelmetallkatalysatoren können mit oder ohne Trägermaterial zur Anwendung kommen. Vorteilhafte Ergebnisse wurden im Fall des nicht geträgerten Katalysators beim Einsatz von Silberkristallen und insbesondere von Silberwolle erzielt. Als Trägermaterial wird bevorzugt ein basisches oder saures Trägermaterial eingesetzt, welches vorzugsweise aus Steatit, Aluminiumoxid, Alumosilikat, Mineralien aus der Gruppe der Hydrotalcite oder deren Mischungen ist. Ein besonders bevorzugtes Trägermaterial ist Steatit, ein keramischer Werkstoff auf der Basis natürlicher Rohstoffe, der aus der Hauptkomponente Speckstein (Mg(Si₄O₁₀)(OH)₂), einem natürlichem Magnesiumsilikat, besteht. Weiterhin können Zusätze von Ton und Feldspat oder Bariumcarbonat enthalten sein.

Im Fall des geträgerten edelmetallhaltigen Katalysators wurden besonders vorteilhafte Ergebnisse erzielt, wenn dieser durch Aufbringen einer komplexierten schwer löslichen Verbindung des Edelmetalls, ggf. in Mischung mit als Promotoren wirkenden Zusätzen, aus Suspension oder Lösung auf ein Trägermaterial aufgebracht wurde und das erhaltene Produkt anschließend thermisch bei Temperaturen im Bereich von 100 bis 400°C über einen Zeitraum von 5 min bis 5 h behandelt wurde.

Durch diese thermische Behandlung wird auf der Oberfläche des Trägermaterials aus der Edelmetallverbindung das Edelmetall selbst gebildet, welches dann die aktive Spezies des geträgerten Katalysators darstellt.

Die Edelmetallgehalte, gemessen in Gew.-% und bezogen auf das Trägermaterial, liegen im allgemeinen im Bereich von 0,1 bis 40 Gew.-%, vorzugsweise im Bereich von 0,5 bis 20 Gew.-% und besonders bevorzugt im Bereich von 1 bis 10 Gew.-%.
Der Promotorgehalt liegt im Allgemeinen im Bereich 0 bis 1000 Gew.-ppm, bezogen auf das Trägermaterial.

Die Verwendung von Silberoxalat als schwer löslicher Edelmetallverbindung ist bevorzugt, wobei die vorherige in situ-Herstellung des Oxalats besonders bevorzugt ist. Ein derartiges Verfahren zur Herstellung eines geträgerten edelmetallhaltigen Katalysators wird in der nicht vorveröffentlichten Anmeldung PCT/EP2009/053081 beschrieben.

Bei dem erfindungsgemäßen Verfahren beträgt die Verweilzeit der Reaktanten in der katalytisch aktiven Zone im allgemeinen 1 bis 1000 ms, vorzugsweise 100 bis 400 ms. Die Temperatur beträgt im Allgemeinen 300 bis 600 °C, vorzugsweise 350 bis 450°C.

Erfindungsgemäß wird mindestens einem oder mehreren der Reaktanten ein Inertgas (z. B. Stickstoff, Kohlendioxid) beigemischt. Das sauerstoffhaltige Gas enthält bevorzugt einen Überschuss an Inertgas. Im allgemeinen beträgt der Sauerstoffanteil des sauerstoffhaltigen Gases 0,1 bis 100 Vol.%, vorzugsweise 0,1-1 Vol.% ,in Relation zu einem Inertgas.

Das molare lokale Verhältnis von Sauerstoff zu Formamid (n_{O2}/n_{FA}) beträgt im Allgemeinen 0,1-20, insbesondere 0,5-5.

Gemäß einer Ausführungsform des erfindungsgemäßen Verfahrens wird mindestens ein Reaktant am Eintritt des Reaktors zugegeben und mindestens ein anderer Reaktant kurz vor der Katalysatorschüttung hinzugegeben. So kann zum Beispiel die Dosierung des sauerstoffhaltigen Gases sowohl zusammen mit der Formamid-Dosierung am Anfang des Rohrreaktors vorgesehen werden, als auch erst kurz vor der Katalysatorschüttung, um einen relativ kurzen Kontakt des Formamids mit dem Sauerstoff zu gewährleisten.

Nach einer anderen Ausführungsform des erfindungsgemäßen Verfahrens wird mindestens ein Reaktant am Eintritt des Reaktors zugegeben und mindestens ein anderer Reaktant an mehreren Stellen über die Reaktorlänge bzw. -höhe verteilt zugegeben. So ist zum Beispiel eine verteilte Dosierung des sauerstoffhaltigen Gases möglich, bei der dieses dem Reaktor an verschiedenen Stellen zugeführt wird, um lokal große Überschüsse an Sauerstoff und die damit verbundene Verbrennung von Kohlenwasserstoffen zu vermeiden. Ist die Reaktionszone des erfindungsgemäßen Verfahrens als katalytische Wirbelschicht ausgeführt, welche Katalysatorpartikel und gegebenenfalls inerte Partikel enthält, entspricht diese Ausführungsform einer verteilten Zugabe über die Höhe der Wirbelschicht.

Die Fluidisierung der Wirbelschicht kann durch das sauerstoffhaltige Gas und/oder ein Inertgas erreicht werden.

Nach Durchströmen der Katalysatorschüttung bzw. der Wirbelschicht werden die Reaktionsgase schnell und intensiv durch direkten Kontakt mit einer alkoholhaltigen Quenchflüssigkeit vermischt, so dass sich die Mischung soweit abkühlt, dass die schwersiedenden Reaktionsprodukte und das verbliebene Edukt auskondensieren und entstandene Isocyanatgruppen mit dem Alkohol zum entsprechenden Urethan abreagieren. Dabei kann es sich um ein Einspritzen oder Versprühen der Quenchflüssigkeit mittels beliebiger gängiger Systeme (z.B. Venturi-Wäscher) als auch ein Einleiten der Reaktionsgase in die Quenchflüssigkeit (z.B. in einer Blasensäule) handeln. Grundsätzlich sind alle Apparate und Konzepte denkbar, die einen intensiven Kontakt zwischen Gas- und Flüssigphase ermöglichen.

Das molare Verhältnis von Quenchflüssigkeit zu Formamid beträgt im Allgemeinen 1-10000, bevorzugt 10-1000.

Durch das Quenchen sollte die entstandene Mischung aus Reaktionsprodukten und Quenchflüssigkeit eine Temperatur deutlich niedriger als die Siedetemperatur der eingesetzten Quenchflüssigkeit aufweisen. Im Allgemeinen sollte die Temperatur der Reaktionsmischung 40 bis 80°C unterhalb der Siedetemperatur der Quenchflüssigkeit liegen, um zu vermeiden, dass Reaktionsprodukte und größere Mengen Quenchflüssigkeit den Quench über die Gasphase verlassen.

Bei der Quenchflüssigkeit handelt es sich um mindestens einen aliphatischen oder aromatischen Mono- oder Polyalkohol mit 1 bis 10 Kohlenstoffatomen, welcher allein oder in Mischung mit mindestens einem aprotischen dipolaren Lösungsmittel eingesetzt werden kann. Der Alkohol ist vorzugsweise ein aliphatischer C₁ -C₆- Alkohol, wie Methanol, Ethanol, n- oder iso-Propanol, n-, iso- und/oder tert-Butanol, insbesondere Isobutanol.

Der Alkohol bzw. die alkoholische Mischung sollte im Verhältnis zu den gebildeten Isocyanaten im deutlichen Überschuss vorliegen. Das molare Verhältnis Alkohol zu Isocyanat beträgt vorzugsweise > 5, besonders bevorzugt > 50. Das für die alkoholische Mischung verwendete aprotische dipolare Lösungsmittel ist vorzugsweise ein primäres und/oder sekundäres Amid wie Dimethylformamid oder Dimethylacetamid. Das molare Verhältnis dieses Lösungsmittels zu dem Alkohol beträgt vorzugsweise 0-20, besonders bevorzugt 0,25 - 5.

Die bei dem Quenchvorgang anfallenden Abgase und eventuell darin enthaltenen Leichtsieder werden vorzugsweise bis auf Raumtemperatur abgekühlt und können anschließend in den Prozess rezykliert werden, wobei ein Purgestrom zur Ausschleusung der gasförmigen Reaktionsprodukte vorgesehen werden sollte.

Die flüssigen Produkte werden nach der Quenchstufe aufgetrennt (z.B. Rektifikation), wobei nicht vollständig abreagierte Formamide in die Reaktionsstufe zurückgeführt werden können. Der für den Quenchvorgang eingesetzte Alkohol und gegebenenfalls das dabei verwendete Lösungsmittel werden ebenfalls abgetrennt und können in die Quenchstufe zurückgeführt werden.

Im Anschluss an den Quenchvorgang kann aus dem gebildeten Urethan das entsprechende Isocyanat durch Spaltung des Urethans freigesetzt werden, wobei der dabei freigesetzte Alkohol in den Prozess zurückgeführt werden kann. Eine solche Spaltung des Urethans zur Synthese von Isocyanaten ist z. B aus DE-A-19855959 bekannt und erfolgt vorzugsweise thermisch bei 200 bis 600°C, bevorzugt 250 bis 450° C, und Drücken von üblicherweise 1 mbar bis 1,5 bar, bevorzugt 1 mbar bis 1 bar. Zur Spaltung des Urethans werden i.a. Kolonnen (EP 0795543 B1), Wirbelschichtreaktoren (EP 555 628 B1, DE 19907648 A1), Fallfilm- oder Dünnschichtverdampfer (EP 0 092 738 B1) verwendet. Die Spaltung kann in der Flüssig- oder in der Gasphase (EP 100047 A1, EP 0 028 724 A1) betrieben werden.

Gemäß einer besonderen Ausführungsform kann das nach dem Quenchprozess entstandene N-Arylurethan vor der Urethanspaltung einer Kupplung mit Formaldehyd zugeführt werden, wobei sich höhermolekulare methylenverknüpfte N-Arylurethane bilden. Die Kupplung von N-Arylurethanen mit Formaldehyd ist bekannt (EP-A 0 123 412, EP-A-0030039, US-B 6,433,219, H.-G- Franck und J.W. Stadelhofer: Industrielle Aromatenchemie, Springer-Verlag, 1987). So wird beispielsweise N-Phenylurethan mit Formaldehyd in Gegenwart eines Katalysators basierend auf einer starken Säure wie z.B. H₂SO₄, BF, Trifluoressigsäure, HCl in einem aromatischen Lösungsmittel wie Benzol, Nitrobenzol bei Temperaturen von 70 bis 100°C umgesetzt. Die Aufarbeitung zum Erhalt des Reaktionsprodukts erfolgt nach gängigen Methoden. Aus dem so entstandenen Produkt kann dann durch die zuvor beschriebene Urethanspaltung polymeres Methylendiaryldiisocyanat, z.B. PMDI, erhalten werden. Daraus können anschließend die entsprechenden Diarylmethandiisocyanate in der gewünschten Reinheit destillativ gewonnen werden. Von besonderer technischer Bedeutung ist die Herstellung von PMDI und die daraus gewonnenen Diphenylmethandiisocyanate.

### Beispiele:

### Herstellung des Trägerkatalysators (Beispiele 3 und 4):

Steatit-Kugeln mit einem Durchmesser von 1,6 bis 2,2 mm (Hersteller Firma Ceram Tec) wurden durch Aufbringen einer mit Ethylendiamin komplexierten Lösung von Silberoxalat und Kupferoxalat benetzt. Die so mit der Silber-Kupferlösung benetzten Steatit-Kugeln wurden anschließend im Luftstrom bei 280°C für 12 Minuten behandelt. Die Edelmetall-Beladung des Katalysators nach der Beschichtung ist in den jeweiligen Beispielen angegeben.

### Beispiel 1

In einem Rohrreaktor wurden 13 ml/h Toluolbisformamid (TBF)-Schmelze bei 455°C zusammen mit Stickstoff als Inertgas kontinuierlich in einer Quarzglasschüttung verdampft, an 100 g Silberwolle mit Sauerstoff umgesetzt und anschließend mit 3,5 l/h Quenchflüssigkeit, bestehend aus 80 Massen% Dimethylacetamid und 20 Massen% Isobutanol, auf 35°C abgekühlt. Dabei betrug die Verweilzeit in der Katalysatorschüttung 307 ms bei einem molaren Sauerstoffüberschuss (n_{O2}/n_{TBF}) von 11. 33 Mol% des TBFs wurden vollständig oxidiert. Der Umsatz an TBF zu aromatischen Produkten betrug 98 Mol%, die Selektivität zur Isocyanatgruppe (ohne Totaloxidation) betrug 55 Mol%.

### Beispiel 2

In einem Rohrreaktor wurden 6 ml/h Toluolbisformamid-Schmelze bei 425°C zusammen mit Stickstoff als Inertgas kontinuierlich in einer Quarzglasschüttung verdampft, an 100 g Silberwolle mit Sauerstoff umgesetzt und anschließend mit 3,5 l/h Quenchflüssigkeit bestehend aus 20 Massen% Dimethylacetamid und 80 Massen% Isobutanol auf 35°C abgekühlt. Dabei betrug die Verweilzeit in der Katalysatorschüttung 245 ms bei einem molaren Sauerstoffüberschuss (n_{O2}/n_{TBF}) von 4. 7 Mol% des TBFs wurden vollständig oxidiert. Der TBF-Umsatz betrug 97 Mol%, die Selektivität zur Isocyanatgruppe (ohne Totaloxidation) betrug 74 Mol%.

### Beispiel 3

In einem Rohrreaktor wurden 7,7 ml/h Toluolbisformamid-Schmelze bei 400°C zusammen mit Stickstoff als Inertgas kontinuierlich in einer Quarzglasschüttung verdampft, an 80 g Silber auf Steatit (12 Gew.-% Ag) mit Sauerstoff umgesetzt und anschließend mit 3,5 l/h Quenchflüssigkeit bestehend aus 20 Massen% Dimethylacetamid und 80 Massen% Isobutanol auf 35°C abgekühlt. Dabei betrug die Verweilzeit in der Katalysatorschüttung 332 ms bei einem molaren Sauerstoffüberschuss (n_{O2}/n_{TBF}) von 3. 5 Mol% des TBFs wurden vollständig oxidiert. Der TBF-Umsatz betrug 75Mol%, die Selektivität zur Isocyanatgruppe (ohne Totaloxidation) betrug 60 Mol%.

### Beispiel 4

In einem Rohrreaktor wurden 10 ml/h Toluolbisformamid-Schmelze bei 450.°C zusammen mit Stickstoff als Inertgas kontinuierlich in einer Quarzglasschüttung verdampft, an 80 g Silber (9,1 Gew.-%)/Cu (150 Gew.-ppm) auf Steatit mit Sauerstoff umgesetzt und anschließend mit 3,5 l/h Quenchflüssigkeit bestehend aus 20 Massen% Dimethylacetamid und 80 Massen% Isobutanol auf 35°C abgekühlt. Dabei betrug die Verweilzeit in der Katalysatorschüttung 310 ms bei einem molaren Sauerstoffüberschuss (n_{O2}/n_{TBF}) von 13. 57 Mol% des TBFs wurden vollständig oxidiert. Der TBF-Umsatz betrug 91 Mol%, die Selektivität zur Isocyanatgruppe (ohne Totaloxidation) betrug dabei 72 Mol%.

### Beispiel 5

In einem Rohrreaktor wurden 27 g/h Formanilid bei 400°C zusammen mit CO₂ als Inertgas kontinuierlich verdampft, an 30 g Silberwolle mit Sauerstoff umgesetzt und anschließend mit Quenchflüssigkeit bestehend aus 100 Massen% Isobutanol auf 35°C abgekühlt. Dabei betrug die Verweilzeit in der Katalysatorschüttung 230 ms bei einem molaren Sauerstoffunterschuss (n_{O2}/n_{TBF}) von 0,37. Der Formanilid-Umsatz betrug hierbei 50 Mol%, die Selektivität zur Isocyanatgruppe betrug 97 Mol%.

## Patentansprüche

1. Verfahren zur Herstellung von aromatischen Isocyanaten durch Umsetzung der korrespondierenden Formamide mit einem sauerstoffhaltigen Gas an Edelmetallkatalysatoren der Metalle Cu, Au, Ag, Re, Pd, Pt, Rh, Ru, W oder Os oder deren Mischungen bei Temperaturen von 300 bis 600 °C und einer Kontaktzeit von 1 bis 1000 ms, **gekennzeichnet durch**:
a. Verdampfung des Formamids vor Eintritt in die Reaktionszone,
b. Quench der erhaltenen Reaktionsmischung mit einer alkoholhaltigen Quenchflüssigkeit und
c. Spaltung des gebildeten Urethans zu Isocyanat und Alkohol.

2. Verfahren nach Anspruch 1, **dadurch gekennzeichnet, dass** es sich bei dem eingesetzten Formamid um ein mono- oder polycyclisches aromatisches Mono-, Bis- oder Polyformamid handelt.

3. Verfahren nach Anspruch 1 oder 2, **dadurch gekennzeichnet, dass** der Edelmetallkatalysator ein Silberkatalysator aus reinem Silber oder ein silberhaltiger Mischkatalysator mit oder ohne Trägermaterial ist.

4. Verfahren nach einem der Ansprüche 1 bis 3, **dadurch gekennzeichnet, dass** es sich bei dem Alkohol der alkoholhaltigen Quenchflüssigkeit um einen alphatischen oder aromatischen Mono- oder Polyalkohol der Kettenlänge C₁-C₁₀ handelt.

5. Verfahren nach Anspruch 4, **dadurch gekennzeichnet, dass** dem Alkohol mindestens ein aprotisch dipolares Lösungsmittel zugesetzt wird.

6. Verfahren nach einem der Ansprüche 1 bis 5, **dadurch gekennzeichnet, dass** das molare Verhältnis von Quenchflüssigkeit zu Formamid 1-10000 beträgt, bevorzugt 10-1000.

7. Verfahren nach einem der Ansprüche 1 bis 6, **dadurch gekennzeichnet, dass** das Quenchen durch Versprühen, Verdüsen oder durch Einleiten der Reaktionsgase in die Quenchflüssigkeit erfolgt.

8. Verfahren nach einem der Ansprüche 1 bis 7, **dadurch gekennzeichnet, dass** die entstandene Mischung aus Reaktionsprodukten und Quenchflüssigkeit eine Temperatur deutlich kleiner als die Siedetemperatur der Quenchflüssigkeit aufweist.

9. Verfahren nach einem der Ansprüche 1 bis 8, **dadurch gekennzeichnet, dass** die entstandene flüssige Produktlösung durch geeignete mechanische und/oder thermische Trennverfahren von der Quenchflüssigkeit, den Ausgangsstoffen sowie unvollständig umgesetzten Reaktanten befreit wird, welche anschließend in das Verfahren zurückgeführt werden.

10. Verfahren nach Anspruch 1, **dadurch gekennzeichnet, dass** die Spaltung des Urethans thermisch bei einer Temperatur im Bereich von 200 bis 600°C und einem Druck im Bereich von 1 mbar bis 1,5 bar erfolgt.

11. Verfahren nach Anspruch 1, **dadurch gekennzeichnet, dass** ein nach dem Quenchprozess entstandenes N-Arylurethan vor der nachfolgenden Spaltung zum Isocyanat zur Bildung von höhermolekularen methylenverknüpften N-Arylurethanen einer Kupplung mit Formaldehyd zugeführt wird.

## Claims

1. A process for preparing aromatic isocyanates by reacting the corresponding formamides with an oxygen-comprising gas over noble metal catalysts comprising the metals Cu, Au, Ag, Re, Pd, Pt, Rh, Ru, W or Os mixtures thereof at temperatures of from 300 to 600°C and a contact time of from 1 to 1000 ms, wherein:
a. the formamide is vaporized before entering the reaction zone,
b. the reaction mixture obtained is quenched with an alcohol-comprising quenching liquid and
c. the urethane formed is dissociated into isocyanate and alcohol.

2. The process according to claim 1, wherein the formamide used is a monocyclic or polycyclic aromatic monoformamide, bisformamide or polyformamide.

3. The process according to claim 1 or 2, wherein the noble metal catalyst is a silver catalyst composed of pure silver or a silver-comprising mixed catalyst with or without support material.

4. The process according to any of claims 1 to 3, wherein the alcohol of the alcohol-comprising quenching liquid is an aliphatic or aromatic monoalcohol or polyalcohol having a chain length of C₁-C₁₀.

5. The process according to claim 4, wherein at least one aprotic dipolar solvent is added to the alcohol.

6. The process according to any of claims 1 to 5, wherein the molar ratio of quenching liquid to formamide is 1-10 000, preferably 10-1000.

7. The process according to any of claims 1 to 6, wherein quenching is effected by spraying, atomizing or by passing the reaction gases into the quenching liquid.

8. The process according to any of claims 1 to 7, wherein the resulting mixture of reaction products and quenching liquid has a temperature significantly below the boiling point of the quenching liquid.

9. The process according to any of claims 1 to 8, wherein the resulting liquid product solution is freed of the quenching liquid, the starting materials and incompletely reacted reactants by means of suitable mechanical and/or thermal separation processes and the incompletely reacted reactants are subsequently recirculated to the process.

10. The process according to claim 1, wherein the dissociation of the urethane is carried out thermally at a temperature in the range from 200 to 600°C and a pressure in the range from 1 mbar to 1.5 bar.

11. The process according to claim 1, wherein an N-arylurethane formed after the quenching process is subjected to coupling with formaldehyde to form relatively high molecular weight methylene-linked N-arylurethanes before the subsequent dissociation to give the isocyanate.

## Revendications

1. Procédé de fabrication d'isocyanates aromatiques par mise en réaction des formamides correspondants avec un gaz contenant de l'oxygène sur des catalyseurs de métaux nobles des métaux Cu, Au, Ag, Re, Pd, Pt, Rh, Ru, W ou Os ou leurs mélanges à des températures de 300 à 600 °C et pendant un temps de contact de 1 à 1 000 ms, **caractérisé par** :
a. une évaporation du formamide avant l'entrée dans la zone de réaction,
b. une désactivation du mélange réactionnel obtenu avec un liquide de désactivation contenant un alcool et
c. un clivage de l'uréthane formé en isocyanate et alcool.

2. Procédé selon la revendication 1, **caractérisé en ce que** le formamide utilisé est un mono-, bis- ou polyformamide aromatique mono- ou polycyclique.

3. Procédé selon la revendication 1 ou 2, **caractérisé en ce que** le catalyseur de métal noble est un catalyseur d'argent constitué d'argent pur ou un catalyseur mixte contenant de l'argent avec ou sans matériau support.

4. Procédé selon l'une quelconque des revendications 1 à 3, **caractérisé en ce que** l'alcool du liquide de désactivation contenant un alcool est un mono- ou polyalcool aliphatique ou aromatique d'une longueur de chaîne en C₁-C₁₀.

5. Procédé selon la revendication 4, **caractérisé en ce qu'**au moins un solvant dipolaire aprotique est ajouté à l'alcool.

6. Procédé selon l'une quelconque des revendications 1 à 5, **caractérisé en ce que** le rapport molaire entre le liquide de désactivation et le formamide est de 1 à 10 000, de préférence de 10 à 1 000.

7. Procédé selon l'une quelconque des revendications 1 à 6, **caractérisé en ce que** la désactivation a lieu par pulvérisation, atomisation ou par introduction des gaz réactionnels dans le liquide de désactivation.

8. Procédé selon l'une quelconque des revendications 1 à 7, **caractérisé en ce que** le mélange formé des produits de réaction et du liquide de désactivation présente une température bien inférieure à la température d'ébullition du liquide de désactivation.

9. Procédé selon l'une quelconque des revendications 1 à 8, **caractérisé en ce que** la solution de produits liquide formée est débarrassée du liquide de désactivation, des matières premières et des réactifs incomplètement réagis par des procédés de séparation mécaniques et/ou thermiques appropriés, puis ceux-ci sont recyclés dans le procédé.

10. Procédé selon la revendication 1, **caractérisé en ce que** le clivage de l'uréthane a lieu thermiquement à une température dans la plage allant de 200 à 600 °C et à une pression dans la plage allant de 1 mbar à 1,5 bar.

11. Procédé selon la revendication 1, **caractérisé en ce qu'**un N-aryluréthane formé après le procédé de désactivation est introduit dans un couplage avec du formaldéhyde avant le clivage ultérieur en l'isocyanate pour la formation de N-aryluréthanes à liaison méthylène de poids moléculaire élevé.
